# EUROPÄISCHE PATENTSCHRIFT

(11) **EP 3 171 826 B1**
(45) Veröffentlichungstag und Bekanntmachung des Hinweises auf die Patenterteilung: **18.12.2019**
(21) Anmeldenummer: 15741122.4
(22) Anmeldetag: 21.07.2015
(51) Int. Cl.: A61F 2/78, A61F 2/80, A61F 2/74

(54) **LINER FÜR EINE PROTHESE**
LINER FOR A PROSTHESIS
GARNITURE POUR PROTHÈSE

(30) Priorität: 22.07.2014 DE 102014010683
(43) Veröffentlichungstag der Anmeldung: 31.05.2017
(73) Patentinhaber: Ottobock SE & Co. KGaA, 37115 Duderstadt (DE)
(72) Erfinder: MÜLLER, André, 37115 Duderstadt (DE); SCHMIDT, Dominik, 78120 Furtwangen (DE)
(74) Vertreter: Lins, Martina
(86) Internationale Anmeldenummer: PCT/EP2015/001501
(87) Internationale Veröffentlichungsnummer: WO 2016/012094

(56) Entgegenhaltungen:
- WO-A1-2010/085336
- US-A- 5 980 577
- US-A1- 2007 055 383
- US-A1- 2008 086 218
- US-A1- 2008 221 706
- US-A1- 2012 191 217
- US-B1- 6 544 292

## Beschreibung

Die Erfindung betrifft einen Liner für eine Prothese, der eine Innenseite und eine Außenseite aufweist, wobei zwischen der Innenseite und der Außenseite wenigstens ein Strömungskanal (16) mit wenigstens einer Einlassöffnung (10) und wenigstens einer Auslassöffnung verläuft, wobei ein Ein-Wege-Ventil (14) derart im Verlauf des wenigstens einen Strömungskanals (16) angeordnet ist, dass dieser nur von der wenigstens einen Einlassöffnung (10) zu der wenigstens einen Auslassöffnung durchströmbar ist.

Ein derartiger Liner ist beispielsweise aus der US 6 544 292 B1 bekannt.

Derartige Liner werden über einen Amputationsstumpf des Patienten gezogen, bevor dieser in einen starren Prothesenschaft eingeführt wird. Aus dem Stand der Technik sind unterschiedliche Möglichkeiten bekannt, wie der Prothesenschaft und damit die Prothese selbst am Amputationsstumpf des Patienten beziehungsweise an dem Liner, der über dem Amputationsstumpf getragen wird, befestigt wird. Weit verbreitet ist die Methode, zwischen dem Liner und dem Prothesenschaft ein möglichst luftdicht abgeschlossenes Volumen zu definieren und innerhalb dieses Volumens einen Unterdruck aufzubauen, der dafür sorgt, dass der Prothesenschaft am Liner festgesaugt wird. Ein solcher Liner ist beispielsweise aus der WO 2009/062489 A1 bekannt. Die Innenseite liegt dabei am Amputationsstumpf an, während die Außenseite beispielsweise mit einem Prothesenschaft in Kontakt steht. Durch die Ausbildung des Liners mit einem Innenliner und einem Außenliner wird die Haftung am Amputationsstumpf und am Prothesenschaft verbessert. Insbesondere kann der proximale Anteil des Außenliners um den proximalen Rand des Prothesenschaftes umgeklappt werden.

Da es aufgrund von Leckagen und kleinen Undichtigkeiten immer zu einem Nachfließen von Luft in das so evakuierte Volumen kommen kann, ist es wichtig, dafür zu sorgen, den Unterdruck in diesem Volumen zwischen Liner und Prothesenschaft beständig aufrecht zu erhalten. Hinzu kommt, dass es für unterschiedliche Bewegungsmodi und Betätigungsformen des Amputationsstumpfes sinnvoll ist, unterschiedlich starke Unterdrücke in dem Volumen zu erzeugen und aufrecht zu erhalten. So ist es beispielsweise nötig, beim Laufen mit einer Unterschenkelprothese einen deutlich stärkeren Unterdruck in dem Volumen zu erzeugen, als dies nötig ist, wenn der Träger der Prothese beispielsweise sitzt.

Aus dem Stand der Technik ist eine Reihe von Möglichkeiten bekannt, den Unterdruck aufrecht zu erhalten. Zunächst wurde das Volumen beispielsweise durch eine elektrisch betriebene Pumpe evakuiert. Dies hat jedoch eine Reihe von Nachteilen. So ist einerseits zusätzliches Gewicht und Volumen für die Pumpe vom Träger der Prothese mit herumzutragen und andererseits muss für die beständige Stromversorgung der Pumpe beispielsweise durch Batterien gesorgt werden. Hinzu kommt, dass die Pumpe zu einer störenden Geräuschentwicklung führt, was den allgemeinen Tragekomfort der Prothese verringert.

Daher wurde die separate elektrisch betriebene Pumpe in vielen Ausführungsformen durch eine mechanische Pumpe ersetzt, bei der die Bewegung des Liners beziehungsweise des in dem Liner befindlichen Amputationsstumpfes genutzt wird, um das Volumen zwischen dem Liner und dem Prothesenschaft zu evakuieren. Derartige Ausführungsformen sind beispielsweise aus der US 8,197,555 B2 und der US 2012/0191217 bekannt. In beiden Dokumenten werden Prothesen beschrieben, bei denen zwischen dem Liner und dem Prothesenschaft im distalen Bereich des Liners ein Volumen vorhanden ist, das im belasteten Zustand, also beispielsweise beim Stehen, zusammengedrückt und komprimiert wird. In dem Volumen befindliche Luft wird auf diese Weise durch ein Ein-Wege-Ventil gedrückt, das sich im Prothesenschaft befindet. Auf diese Weise kommt es zu einer Druckverringerung im Inneren des Volumens und somit zur Aufrechterhaltung des Unterdrucks.

Aus der DE 10 2006 054 891 A1 ist ein becherförmiger Prothesenschaft bekannt, bei dem im posterioren Bereich eine aus Folien gebildete flexible Kammer im Innenraum des Prothesenschaftes angeordnet ist. Auch diese wird bei Gehbewegungen zusammengedrückt, so dass in der Kammer enthaltene Luft durch eine dafür vorgesehene durch den Prothesenschaft hindurch geführte Auslassleitung und ein daran angeordnetes Ein-Wege-Ventil aus dem Prothesenschaft herausgepresst wird. Anders als bei den zuvor genannten Ausführungsformen ist hier die Pumpkammer jedoch nicht im distalen Bereich des Amputationsstumpfes angeordnet.

Die Funktionsweise einer derartigen Pumpe, die die Bewegung des Amputationsstumpfes nutzt, um ein Volumen zu evakuieren, ist beispielsweise in der EP 1 771 659 B1 beschrieben. Im Innern der Pumpkammer befindet sich beispielsweise ein elastisch verformbares Material, das die Rückstellkraft aufbringt und so dafür sorgt, dass die Pumpkammer sich im entlasteten Zustand wieder entfaltet und Luft aus dem zu evakuierenden Volumen aufnimmt.

Aus der US 8,357,206 B2 und der US 2012/0191218 A1 ist jeweils ein Liner bekannt, der mit einer Verteil- oder Leitungslage an der Außenseite des Liners versehen ist. Diese besteht aus einem porösen und gasleitenden Material und bildet auf diese Weise eine über den nahezu gesamten Amputationsstumpf verteilte Pumpkammer. Alternativ kann auch eine separate externe Pumpe angeschlossen werden, die Luft und gegebenenfalls Feuchtigkeit aus der Verteil- oder Leitungslage absaugt. In der US 2012/0191218 A1 ist der Liner im distalen Bereich des Amputationsstumpfes zudem porös beziehungsweise mit einer Vielzahl von Löchern und Durchlässen versehen, um auf diese Weise Flüssigkeit vom Amputationsstumpf abzuleiten.

Auch in diesen Ausführungsformen wird jedoch ein Auslassventil und ein Auslasskanal benötigt, der durch den Prothesenschaft hindurch geführt wird, um die Luft aus dem zu evakuierenden Volumen zwischen Liner und Prothesenschaft hinaus zu befördern.

Die Ein-Wege-Ventile und Auslassleitungen oder -kanäle, die zum Abführen der durch die Pumpe aus dem Volumen entfernten Luft nötig sind, sind in aller Regel im distalen Bereich des Prothesenschaftes angeordnet, dessen Aufbauhöhe sich dadurch erhöht und dessen Konstruktion dadurch verkompliziert wird. Da der Prothesenschaft individuell an den Träger der Prothese angepasst wird, ist es wünschenswert, für den Orthopädietechniker, der diese Anpassung vornimmt, einen ansonsten möglichst einfachen Aufbau des Prothesenschaftes bereitstellen zu können. Insbesondere bei Amputationen beispielsweise kurz oberhalb des Knies eines Beines, ist es nötig, dass sich direkt an das distale Ende des Prothesenschaftes das Prothesenknie anschließt, so dass in diesen Fällen der Platz für eine aufwändige Ventilanordnung nicht ausreichend ist.

Aus der US 2007/0055383 A1 ist ein Linersystem für eine Prothese bekannt, bei dem zwischen dem Liner und dem Amputationsstumpf ein Material, das Schweiß transportieren kann, angeordnet ist. Der Liner verfügt über eine Durchleitung, durch die ein Vakuum zwischen dem Liner und dem Amputationsstumpf angelegt werden kann. Aus der US 5,980,577 hingegen ist ein Prothesensystem bekannt, bei dem Luft zwischen dem Innenschacht und dem Amputationsstumpf eingebracht werden kann.

Der Erfindung liegt daher die Aufgabe zugrunde, einen Liner für eine Prothese vorzuschlagen, mit dem die Nachteile aus dem Stand der Technik behoben werden können.

Die Erfindung löst die gestellte Aufgabe durch einen Liner für eine Prothese gemäß dem unabhängigen Anspruch 1 und weiteren besonderen Ausführungsformen entsprechend den Unteransprüchen, wobei der Liner eine Innenseite und eine Außenseite aufweist gemäß dem Oberbegriff des Anspruchs 1 und sich dadurch auszeichnet, dass zwischen der Innenseite und der Außenseite wenigstens eine Pumpkammer in Fluidverbindung mit dem wenigstens einen Strömungskanal angeordnet ist. Zwischen der Innenseite und der Außenseite wenigstens ein Strömungskanal mit wenigstens einer Einlassöffnung und wenigstens einer Auslassöffnung verläuft, wobei ein Ein-Wege-Ventil derart im Verlauf des wenigstens einen Strömungskanals angeordnet ist, dass dieser nur von der wenigstens einen Einlassöffnung zu der wenigstens einen Auslassöffnung durchströmbar ist. Auf diese Weise lässt sich beispielsweise ein Volumen zwischen dem distalen Ende des Liners und dem Prothesenschaft der Prothese einfach evakuieren, indem die Bewegung des Amputationsstumpfes benutzt wird, ohne dass Ein-Wege-Ventile und Strömungskanäle im distalen Bereich des Prothesenschaftes angeordnet werden müssen. Auch eine Durchführung durch den Prothesenschaft ist nicht mehr nötig, sodass insbesondere die komplizierte Abdichtung zum Aufrechterhalten des Vakuums im Bereich zwischen dem Liner und dem Prothesenschaft nicht mehr erforderlich ist. Dadurch wird der Prothesenschaft konstruktiv vereinfacht, so dass auch die Herstellungskosten sinken.

Zudem lässt sich alles, was zum Evakuieren des Volumens zwischen dem Liner gemäß der vorliegenden Erfindung und einem darüber getragenen Prothesenschaft nötig ist, in dem Liner selbst zwischen der Innenseite und der Außenseite anordnen. Es sind folglich keinerlei Aufbauten oder Vorrichtungen nötig, die in dem Prothesenschaft integriert oder eingebaut werden müssten.

Die Einlassöffnung des Strömungskanals stellt eine Verbindung der Pumpkammer mit dem zu evakuierenden Volumen her. Vorzugsweise bildet die wenigstens eine Pumpkammer ein distales Ende des wenigstens einen Strömungskanals, so dass die Einlassöffnung vorzugsweise direkt in die Pumpkammer mündet. Im belasteten Zustand des Liners, beispielsweise beim Stehen mit einer Oberschenkelprothese, wird die Pumpkammer komprimiert. Vollzieht der Prothesenträger nun einen Gangzyklus, hebt sich der distale Bereich des Liners von einem distalen Bereich der Schaftwand in der Schwungphase ab. Durch die Einlassöffnung des Strömungskanals strömt in diesem Fall Luft, die sich in dem zu evakuierenden Volumen befindet, in die Pumpkammer zwischen Innenseite und Außenseite des Liners. In der folgenden Standphase wird die Einlassöffnung von der Schaftwand des Prothesenschaftes, gegen die sie nun gedrückt wird, luftdicht abgeschlossen, so dass die Luft, die sich in der Pumpkammer befindet, durch die Einlassöffnung nicht entweichen kann. Als Pumpkammer kann jedoch auch ein Bereich zwischen dem Liner und dem Prothesenschaft verwendet werden.

Beim Komprimieren der Pumpkammer tritt die sich in der Pumpkammer befindende Luft durch den Strömungskanal und die Auslassöffnung aus, der wie die Pumpkammer zwischen der Innenseite und der Außenseite des Liners angeordnet ist. Am der Pumpkammer entgegengesetzten Ende des Strömungskanals, das sich vorteilhafterweise am proximalen Rand des Liners befindet, kann die Luft den Liner verlassen. Vollzieht der Prothesenträger nun weitere Gangzyklen, wird mit jedem Schritt die Luft zwischen dem Schaft und dem Liner aus dem dazwischen liegenden Volumen evakuiert. Es entsteht ein aktives Vakuum und somit die gewünschte Haltekraft zwischen dem Amputationsstumpf und dem Prothesenschaft. Die Menge der aus dem Volumen abgezogenen Luft wird bei jedem Schritt geringer, bis sich ein Unterdruckniveau in dem Volumen eingeregelt hat, bei dem ein Ablösen des Liners, der über den Amputationsstumpf gezogen ist, von der Schaftinnenwand des Prothesenschaftes verhindert wird. Auf diese Weise kommt es nicht zu dem aus dem Stand der Technik bekannten Pump- oder Melkeffekt. Auch dies kann mit oder ohne in den Liner integrierter Pumpkammer erreicht werden.

Der erfindungsgemäße Liner kann vom Prothesenträger einfach über den Amputationsstumpf gerollt werden. Anschließend steigt der Prothesenträger in den Prothesenschaft ein. Aufgrund der bereits beschriebenen Funktionsweise des erfindungsgemäßen Liners ist es nicht nötig, zunächst beispielsweise über eine separate externe Pumpe ein Vorvakuum beziehungsweise einen Startunterdruck in dem Volumen zwischen dem Liner und dem Prothesenschaft einzustellen.

Erfindungsgemäß ist der wenigstens eine Strömungskanal mit einem Ein-Wege-Ventil verbunden, das ein Einströmen von Luft durch die wenigstens eine Auslassöffnung des Strömungskanals verhindert. Auf diese Weise wird die Effizienz weiter erhöht, da insbesondere beim Expandieren des Bereichs zwischen dem Liner und dem Prothesenschaft oder der Pumpkammer, bei dem aus dem zu evakuierendem Volumen Luft durch die Einlassöffnung in die Pumpkammer oder den Strömungskanal dringen soll, verhindert wird, dass zusätzlich Luft auch durch die Auslassöffnung in den Strömungskanal hineinströmt.

Als besonders vorteilhaft hat sich dabei herausgestellt, wenn das Ein-Wege-Ventil ebenfalls zwischen der Innenseite und der Außenseite des Liners angeordnet ist. Auch in dieser Ausgestaltung ist folglich alles was zum Evakuieren des Volumens zwischen dem Prothesenschaft und dem Liner nötig ist, zwischen der Innenseite und der Außenseite angeordnet, so dass es nicht nötig ist, separate zusätzliche Bauteile am Prothesenschaft vorzusehen.

Vorteilhafterweise weist der Liner wenigstens abschnittsweise einen Innenliner und einen Außenliner auf. Diese sind teilweise oder vollflächig aneinander angeordnet und bilden vorzugsweise gemeinsam den Liner. Die Innenseite des Liners wird dabei durch die Innenseite des Innenliners gebildet, während die Außenseite des Liners von der Außenseite des Außenliners gebildet wird. Als besonders vorteilhaft hat sich dabei herausgestellt, wenn der Strömungskanal und gegebenenfalls auch die Pumpkammer zwischen dem Innenliner und dem Außenliner angeordnet ist. Dadurch wird der Liner auf besonders einfache Weise herstellbar.

Vorzugsweise bestehen der Innenliner und/oder der Außenliner aus Polyurethan oder Silikon. Als besonders vorteilhaft hat sich dabei herausgestellt, wenn der Innenliner aus Polyurethan gebildet wird, während der Außenliner aus Silikon besteht.

In einer vorteilhaften Ausgestaltung der vorliegenden Erfindung ist das Ein-Wege-Ventil als Flatterventil ausgebildet. Ein Flatterventil ist dabei ein Ein-Wege-Ventil, das ohne sonstigen äußeren Antrieb öffnet und auch selbsttätig wieder schließt. Das Öffnen und Schließen geschieht ausschließlich aufgrund von Druckunterschieden auf den beiden Ventilseiten. Derartige Ventile können sehr kompakt und klein ausgeführt werden und sind wartungsarm und kaum fehleranfällig. Zudem bietet sich diese Art von Ventil insbesondere in einem Liner, der ständig elastischen Verformungen unterworfen ist, an.

Vorzugsweise ist das Ein-Wege-Ventil aus zwei Linerschichten, insbesondere aus dem Innenliner und dem Außenliner, gebildet. Dies kann beispielsweise dadurch geschehen, dass Innenliner und Außenliner im Bereich, der das Ein-Wege-Ventil bilden soll, nicht miteinander verklebt oder in anderer Weise dauerhaft verbunden werden, so dass sich hier ein möglicher Strömungsweg bildet, durch den Luft hindurchtreten kann. Im belasteten Zustand der Prothese, für die der Liner verwendet wird, ist der Luftdruck am distalen Ende des durch die beiden Linerschichten gebildeten Ventils größer als am proximalen Ende, so dass die Luft durch das Ein-Wege-Ventil hindurchgedrückt wird. In der Entlastungsphase kann jedoch Luft nicht auf umgekehrten Wege durch das so gebildete Ein-Wege-Ventil strömen, da der Innenliner und der Außenliner durch den herrschenden Umgebungsdruck und gegebenenfalls durch von dem Prothesenschaft ausgeübten Druck aneinander gepresst werden. Dieser Effekt kann durch beispielsweise in der Pumpkammer herrschenden oder in der Entlastungsphase erzeugten Unterdruck verstärkt werden.

Es ist jedoch nicht notwendig, dass Ein-Wege-Ventil durch den Innenliner und den Außenliner auszubilden. Alternativ dazu kann beispielsweise auch nur in dem Bereich, in dem das Ventil vorgesehen ist, eine zusätzliche Linerschicht vorhanden sein, die die gewünschten Funktionen erfüllt.

In einer weiteren möglichen Ausführungsform wird das Ein-Wege-Ventil durch eine einzige separate Linerschicht gebildet, die aus einem luftdurchlässigen Material besteht und somit den Strömungsweg durch das Ein-Wege-Ventil bildet. Dieses Material kann als zusätzliche Linerschicht zwischen ohnehin vorhandene Schichten eingesetzt werden und so beispielsweise zwischen Innenliner und Außenliner angeordnet sein. Es ist jedoch auch möglich, dieses Material in ein Linermaterial eines ansonsten beispielsweise einstückigen Liners einzugießen. Auch in diesem Fall wird im belasteten Zustand der Prothese Luft durch den in der Pumpkammer herrschenden hohen Luftdruck durch das als Ventil wirkende Linermaterial gedrückt, während der herrschende Umgebungsdruck und gegebenenfalls der durch den Prothesenschaft aufgebrachte Druck dafür sorgt, dass ein Rückströmen von Luft in entgegengesetzter Richtung verhindert wird.

Vorteilhafterweise wird ein Ventilschnabel des Flatterventils aus mehreren, insbesondere zwei, Folienelementen gebildet. In der Expansionsphase der Pumpkammer, in der Luft aus dem zu evakuierenden Volumen durch die Einlassöffnung in den Strömungskanal strömt, ist der Luftdruck auf der der Einlassöffnung zugewandten Seite des Ein-Wege-Ventils kleiner als auf der der Einlassöffnung abgewandten Seite, so dass das Ventil geschlossen ist. Die den Ventilschnabel bildenden Folienelemente liegen dann aneinander an und werden von dem auf der Außenseite, die der Einlassöffnung abgewandt ist, herrschenden höheren Luftdruck zusammengedrückt. Ein Eindringen von Luft ist in diesem Zustand nicht möglich.

Alternativ dazu kann der Ventilschnabel des Flatterventils auch aus nur einem einzigen Folienelement und beispielsweise dem Linermaterial gebildet werden. Dadurch nimmt die Anzahl der benötigten Bauteile ab, so dass die Konstruktion vereinfacht und die Herstellungskosten gesenkt werden.

Zudem ist es möglich, ein weiteres Ein-Wege-Ventil an oder in der Einlassöffnung vorzusehen, wenn diese beispielsweise zum Innenraum zwischen dem Liner und dem Prothesenschaft führt. Auf diese Weise lässt sich die Pumpwirkung verbessern. Auf diese Weise wird auch für den Fall die Einlassöffnung vollständig abgedichtet, indem dies nicht durch den Prothesenschaft selbst geschieht. Auch in diesem Fall wird folglich verhindert, dass Luft in das Pumpvolumen zurückströmen kann. Dies ist beispielsweise für den Fall von Vorteil, in dem der Patient einen Stumpfstrumpf verwendet, der über dem Liner getragen werden kann. Auf diese Weise wird auch in diesem Fall die Pumpleistung nicht beeinträchtigt.

In der Kompressionsphase hingegen, in der die Pumpkammer oder ein Bereich zwischen Liner und Prothesenschaft aufgrund der äußeren Belastungen komprimiert wird, ist der Luftdruck auf der der Einlassöffnung zugewandten Seite des Ein-Wege-Ventils größer als auf der der Auslassöffnung zugewandten Seite, so dass das Ein-Wege-Ventil öffnet. Die den Ventilschnabel bildenden Folienelemente werden auseinander gedrückt und die Luft kann aus der Pumpkammer durch das Ein-Wege-Ventil entweichen.

In einer besonders einfachen Ausgestaltung der vorliegenden Erfindung ist die wenigstens eine Einlassöffnung ein Loch in dem Außenliner. Da sich der Strömungskanal und gegebenenfalls die Pumpkammer in diesem Fall beispielsweise zwischen dem Innenliner und dem Außenliner befindet, wird durch ein Loch im Außenliner eine Verbindung zwischen dem Strömungskanal und falls vorhanden der Pumpkammer und dem Volumen außerhalb des Liners, das sich folglich im angelegten Zustand der Prothese zwischen dem Liner und dem Prothesenschaft befindet, hergestellt. Als vorteilhaft hat sich zudem herausgestellt, wenn für den Fall, dass sich eine Pumpkammer im Liner befindet, auch an der wenigstens einen Einlassöffnung ein entsprechendes Ein-Wege-Ventil angeordnet ist, das einerseits erlaubt, dass Luft durch die Einlassöffnung in die Pumpkammer hineinströmt, das andererseits jedoch verhindert, dass in der Phase, in der die Pumpkammer komprimiert wird, Luft durch die Einlassöffnung die Pumpkammer verlässt. Ein derartiges zusätzliches Ein-Wege-Ventil ist insbesondere dann sinnvoll, wenn nicht oder nicht zuverlässig gewährleistet werden kann, dass die wenigstens eine Einlassöffnung in der Phase, in der die Pumpkammer komprimiert wird, sicher von der Schaftinnenwand luftdicht abgeschlossen wird.

Vorzugsweise verfügt die wenigstens eine Pumpkammer über eine Umfangswandung aus einem elastisch verformbaren Material. Dies hat zur Folge, dass das Material nach dem Komprimieren der Pumpkammer durch die Einwirkung einer äußeren Kraft eine Gegen- oder Rückstellkraft aufbringt, die nach dem Wegfall der äußeren Kraft dafür sorgt, dass die Pumpkammer wieder expandiert und so Luft einsaugt. Die Umfangswandung der Pumpkammer ist dabei vorteilhafterweise aus dem Innenliner und dem Außenliner gebildet. Dies hat eine besonders einfache Ausgestaltung der Pumpkammer zur Folge.

Zusätzlich oder alternativ dazu befindet sich in der Pumpkammer vorteilhafterweise ein elastisch verformbares Material, insbesondere ein offenporiges Material. Auch auf diese Weise wird gewährleistet, dass eine Rückstellkraft auf die Pumpkammer ausgeübt wird, die die Pumpkammer in ihre ursprüngliche Größe expandiert, sobald eine von außen wirkende die Pumpkammer komprimierende Kraft wegfällt. Das elastisch verformbare Material kann in Streifen oder Trennwänden angeordnet sein, so dass zwischen den einzelnen Elementen des elastisch verformbaren Materials ausreichend Raum bleibt, um Luft aufzunehmen. Das elastisch verformbare Material kann jedoch auch offenporig oder porös ausgebildet sein und somit selbst Luft aufnehmen. Ein derartiges Material kann beispielsweise ein offenporiger Schaum oder eine 3D-Matrix, beispielsweise in Form eines 3D-Gewirkes, sein. In diesem Fall kann die gesamte Pumpkammer mit dem elastisch verformbaren Material ausgefüllt werden. Dadurch wird die aufgebrachte Rückstellkraft maximiert und der Pumpeffekt optimiert.

Vorteilhafterweise sind der Innenliner und der Außenliner in einem proximalen Bereich des Liners so aneinander angeordnet, dass der Außenliner separat vom Innenliner umklappbar ist. Innen- und Außenliner können auf unterschiedlichste Arten und Weisen aneinander festgelegt und miteinander verbunden werden. So können Sie beispielsweise in zwei separaten Tauchschritten aneinander angeordnet werden, indem ein Rohling oder ein Formling in ein Bad aus flüssigem Linermaterial getaucht wird. Alternativ dazu ist es auch möglich, zunächst den Innenliner und den Außenliner separat herzustellen und diese anschließend beispielsweise über eine Klebverbindung aneinander zu befestigen. In der beschriebenen vorteilhaften Ausgestaltung sind der Innenliner und der Außenliner im proximalen Bereich des Liners nicht miteinander verbunden, so dass der Außenliner separat vom Innenliner umgeklappt werden kann. Dies hat mehrere Vorteile.

Wird über einen derartigen Liner ein Prothesenschaft gezogen, kann der Außenliner separat vom Innenliner über den proximalen Rand des Prothesenschaftes geklappt werden. Auf diese Weise entsteht einerseits eine luftdichte Verbindung, so dass das Volumen zwischen dem Liner und dem Prothesenschaft abgedichtet wird. Andererseits ist es auf diese Weise möglich, dass zwischen dem Innenliner und dem Außenliner liegende Ein-Wege-Ventil oder die Auslassöffnung des Strömungskanals so freizulegen, dass Luft an dieser Stelle aus dem Liner austreten kann. Wird der Bereich, in dem der Innenliner und der Außenliner im proximalen Bereich des Liners nicht aneinander festgelegt werden, großzügig genug bemessen, lässt sich auf diese Weise ein Liner für unterschiedlichste Stumpflängen der Amputationsstümpfe verwenden.

Durch die vorliegende Erfindung wird folglich ein Liner vorgeschlagen, der einen Strömungskanal sowie ein Verriegelungssystem als integralen Bestandteil des Liners umfasst. Das Evakuieren des Volumens zwischen dem Liner und dem Prothesenschaft und der dadurch erreichte Aufbau der Haltekraft werden durch die Relativbewegung des Liners relativ zum Prothesenschaft beispielsweise beim Gehen erreicht. Die auf diese Weise aus dem Volumen evakuierte Luft kann auf der Trennkante der beiden Linerschichten im proximalen Bereich des Liners ausgestoßen werden. Damit ist ein Evakuieren des Volumens zwischen dem Liner und dem Prothesenschaft möglich, ohne dass in dem Schaft Ausstoßventile, Rückschlagventile oder sonstige Vorrichtungen vorhanden sein müssen.

In einer bevorzugten Ausgestaltung befindet sich die Einlassöffnung distal und/oder die Auslassöffnung proximal am Liner. Auch dadurch wird der Aufbau erleichtert und gleichzeitig erreicht, dass die benötigte Haltekraft sicher und reproduzierbar aufgebracht werden kann.

Vorzugsweise ist der Liner an seiner Außenseite mit einem Textil, einer Faser, einem Gewebe oder einer Oberflächenstruktur versehen. Dadurch wird gewährleistet, dass der angelegte Unterdruck im gesamten Zwischenraum zwischen dem Liner und dem Prothesenschaft wirkt und sich darin "verteilen" kann. Es wird verhindert, dass der Liner in Abschnitten vollflächig am Schaft anliegt und so eine Ausbreitung des Unterdruckes gegebenenfalls verhindert.

Vorteilhafterweise sind an der Außenseite des Liners eine oder mehrere Dichtlippen vorhanden. Diese sind vorzugsweise in radialer Richtung an den Liner angeformt, angeklebt, aufgeschoben oder auf sonstige Weise an dem Liner befestigt. Die Dichtlippen stehen in radialer Richtung über den äußeren Umfang des Liners heraus und sorgen im angelegten Zustand für einen dichtenden Abschluss zur Innenseite eines Prothesenschaftes. Dabei können die Dichtlippen unterschiedlich geformt und in unterschiedlichen Anzahlen vorhanden sein. Oftmals ist es ausreichend, eine radial umlaufende Dichtlippe vorzusehen, während bei anderen Ausführungsformen zwei oder mehr Dichtlippen vorhanden sein müssen. Die tatsächliche Anzahl, Formgebung und Position der Dichtlippen an der Außenseite des Liners hängt von der individuellen Gegebenheit des Amputationsstumpfes sowie der voraussichtlichen Bewegungsprofile und anderen individuellen Eigenschaften und Anforderungen des Trägers des Liners ab.

Durch einen hier beschriebenen Liner kann der Zwischenraum zwischen dem Liner und dem Prothesenschaft evakuiert und das darin enthaltende Vakuum oder der Unterdruck aufrechterhalten werden. Für den Fall, dass der Prothesenschaft ausgezogen, also der Amputationsstumpf mit dem sich darüber befindenden Liner aus dem Prothesenschaft entfernt werden soll, muss das Volumen zwischen der Innenseite des Prothesenschaftes und dem Liner belüftet werden. Dazu ist herkömmlicherweise im Prothesenschaft ein Ventil vorhanden, das betätigt werden kann, so dass der Zwischenraum belüftet wird und ein Aussteigen aus dem Prothesenschaft leicht möglich wird.

In einer bevorzugten Ausgestaltung eines Liners gemäß der vorliegenden Erfindung ist das Ein-Wege-Ventil, das sich im Liner befindet, schaltbar. Dies bedeutet, dass es in einen ersten Zustand und in einen zweiten Zustand bringbar ist, wobei es im ersten Zustand als bereits beschriebenes Ein-Wege-Ventil wirkt und im zweiten Zustand als Zwei-Wege-Ventil verwendet werden kann. Dies bedeutet, dass in diesem Zustand auch Luft von außen in den Zwischenraum zwischen dem Prothesenschaft und dem Liner eindringen kann, so dass der Zwischenraum belüftet und so ein Aussteigen aus dem Prothesenschaft ermöglicht werden kann. Auf diese Weise müssen im Prothesenschaft selbst keine Ventile mehr vorhanden sein, was die Herstellung stark vereinfacht und die Herstellungskosten senkt.

Mit Hilfe der nachfolgenden Zeichnungen wird ein Ausführungsbeispiel der vorliegenden Erfindung näher erläutert. Es zeigt
- Figur 1: - einen Liner gemäß einem ersten Ausführungsbeispiel der vorliegenden Erfindung,
- Figur 2: - den Liner aus Figur 1 in einer Schnittdarstellung,
- Figur 3: - die schematische Schnittdarstellung durch ein Ein-Wege-Ventil für einen Liner gemäß einem weiteren Ausführungsbeispiel der vorliegenden Erfindung,
- Figur 4: - die schematische Darstellung eines Innenliners für einen Liner gemäß einem weiteren Ausführungsbeispiel der vorliegenden Erfindung,
- Figur 5: - die schematische Schnittdarstellung durch einen Liner gemäß einem weiteren Ausführungsbeispiel der vorliegenden Erfindung,
- Figur 6: - den Ausschnitt aus einem weiteren Liner in einer Schnittdarstellung,
- Figur 7: - einen weiteren Liner in einer schematischen Schnittdarstellung,
- Figur 8: - einen vergrößerten Ausschnitt aus Figur 7,
- Figur 9: - einen weiteren Liner in einer schematischen Schnittdarstellung und
- Figur 10 -: einen weiteren Liner in einer schematischen Schnittdarstellung.
- Figuren 11 bis 14 -: weitere Liner in schematischen Schnittdarstellungen.

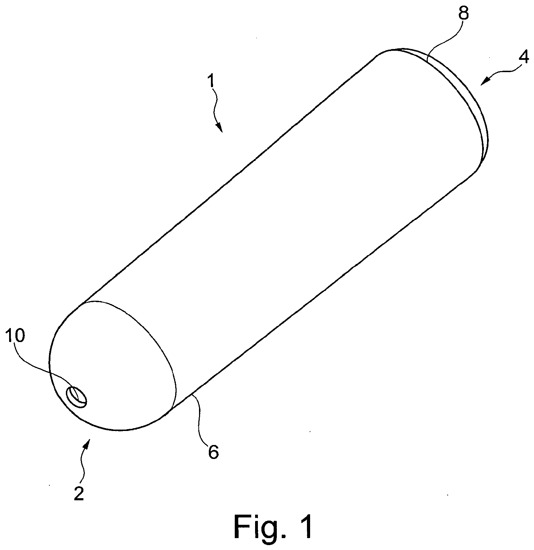

Figur 1 zeigt einen Liner 1, der über ein geschlossenes distales Ende 2 und ein offenes proximales Ende 4 verfügt. Der Liner 1 weist einen Außenliner 6 und einen darin angeordneten Innenliner 8 auf. Am distalen Ende 2 des Außenliners 6 ist eine Einlassöffnung 10 dargestellt, durch die Luft in eine zwischen dem Innenliner 8 und dem Außenliner 6 angeordnete in Figur 1 nicht dar-gestellte Pumpkammer oder einen Strömungskanal strömen kann.

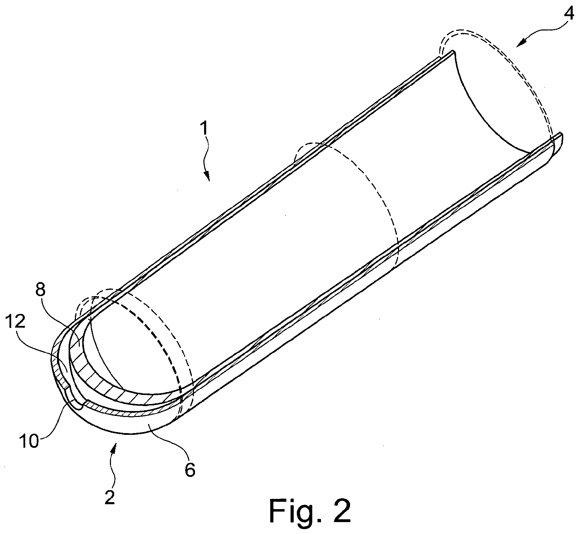

Figur 2 zeigt den Liner 1 aus Figur 1 in einer Schnittdarstellung. Man erkennt im Bereich des distalen Endes 2 zwischen dem Innenliner 8 und dem Außenliner 6 eine Pumpkammer 12, die über die Einlassöffnung 10 mit dem den Liner 1 umgebenden Außenraum verbunden ist.

Zwischen dem Innenliner 8 und dem Außenliner 6 verläuft ein in Figur 2 nicht gezeigter Strömungskanal, der über eine Auslassöffnung mit der Pumpkammer 12 verbunden ist und von der Pumpkammer 12 in Richtung auf das proximale Ende 4 des Liners 1 verläuft. Beispielsweise beim Stehen wird in einem in Figur 2 gezeigten Liner 1 die Pumpkammer 12 komprimiert, da die beim Stehen wirkenden Kräfte die Pumpkammer 12 zusammendrücken. Dabei liegt die Einlassöffnung 10 an einer Innenwand des nicht gezeigten Prothesenschaftes an und wird durch diesen verschlossen. Kann dies nicht oder nicht sicher gewährleistet werden, ist es sinnvoll, an der Einlassöffnung 10 ein Ein-Wege-Ventil vorzusehen, das ein Ausströmen von Luft durch die Einlassöffnung 10 verhindert. Die Luft strömt in diesem Fall durch die nicht gezeigte Auslass-öffnung und den Strömungskanal und verlässt so die Pumpkammer 12.

Bei einem Liner 1 gemäß einem anderen Ausführungsbeispiel nicht gemäß der Erfindung ist zwischen dem Innenliner 8 und dem Außenliner 6 keine Pumpkammer 12 angeordnet. Stattdessen handelt es sich bei der Einlassöffnung 10 um eine Öffnung des in Figur 2 nicht dargestellten Strömungskanals. Die Pumpkammer befindet sich in diesem Fall zwischen dem Liner und dem Prothesenschaft, wird jedoch auf die gleiche Weise evakuiert.

In der Schwungphase, bei der die externe auf die Pumpkammer 12 wirkende Kraft nicht mehr vorhanden ist, expandiert die Pumpkammer 12 aufgrund beispielsweise von Rückstellungskräften eines elastischen Materials, das in der Pumpkammer 12 angeordnet ist. Dadurch wird Luft durch die Einlassöffnung 10 in die Pumpkammer 12 eingesogen und das Volumen, das zwischen dem Liner 1 und dem umgebenden Prothesenschaft eingeschlossen ist, evakuiert.

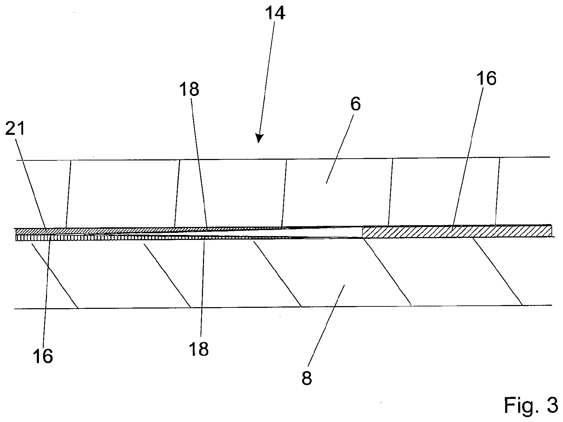

Figur 3 zeigt eine Schnittdarstellung durch ein Ein-Wege-Ventil 14, das im vorliegenden Fall als Flatterventil ausgebildet ist. Zwischen dem Innenliner 8 und dem Außenliner 6 ist ein Strömungskanal 16 angeordnet, der beispielsweise in Form eines zwischen die beiden Linerschichten geklebten Schlauches ausgebildet sein kann. Im in Figur 3 gezeigten Ausführungsbeispiel besteht der Strömungskanal 16 jedoch aus einem luftdurchlässigen Material.

In Figur 3 links dieses Strömungskanals 16 schließt sich das eigentliche Ein-Wege-Ventil 14 an, das im vorliegenden Beispiel einen Ventilschnabel aufweist, der aus zwei Folienelementen 18 besteht. Diese sind beispielsweise über eine Kleberschicht mit dem Innenliner 8 beziehungsweise dem Außenliner 6 verbunden. Links des Ein-Wege-Ventils 14 schließt sich ein weiterer Teil des Strömungskanals 16 an, der im gezeigten Ausführungsbeispiel als poröses luftdurchlässiges Material 21 ausgebildet ist.

Wird beispielsweise beim Gehen in der Pumpkammer 12 ein Überdruck aufgebaut, ist der Luftdruck auf der rechten Seite des in Figur 3 gezeigten Ein-Wege-Ventils 14 größer als auf der linken Seite, so dass Luft durch das Ein-Wege-Ventil 14 gedrückt wird und die beiden Folienelemente 18 öffnet. Luft kann auf diese Weise durch das Ein-Wege-Ventil 14 ausströmen. Anschließend verschließt der anliegende Außendruck die beiden Folienelemente 18 und das Ein-Wege-Ventil 14 ist geschlossen, so dass keine Luft von außen in die Pumpkammer 12 eindringen kann.

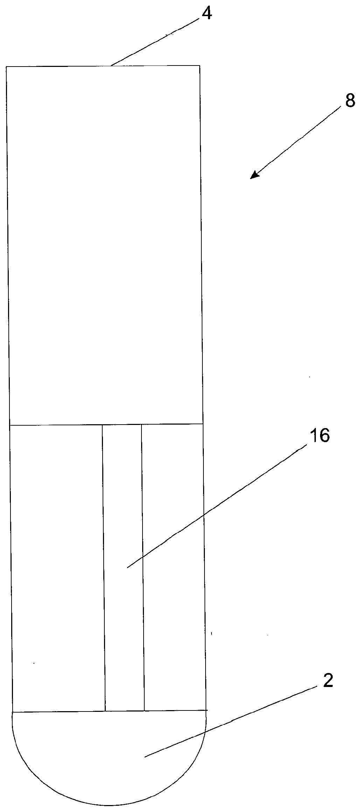

Figur 4 zeigt den Innenliner 8 in einer schematischen Darstellung. Man erkennt an der Außenseite des Innenliners 8 den Strömungskanal 16, in dem sich unter anderem später auch das Ein-Wege-Ventil 14 befinden wird. Der Auslasskanal 16 erstreckt sich bis zum distalen Ende 2 und endet im mittleren Bereich in Richtung auf das proximale Ende 4. Prinzipiell ist es nicht nötig einen voll-flächigen Außenliner 6 zu verwenden. Es ist ausreichend wenn ein Außenliner 6 verwendet wird, der den Bereich des Strömungskanals 16 abdeckt.

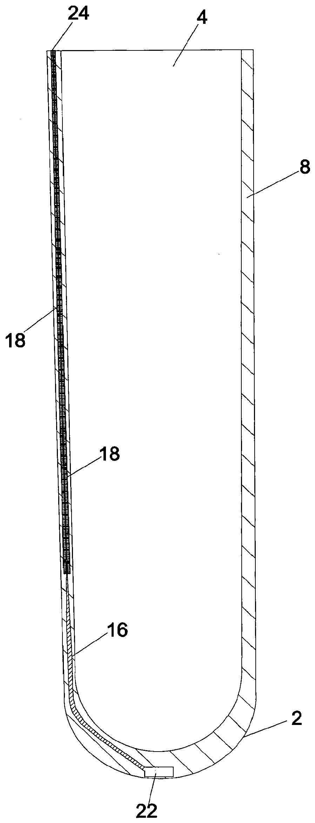

Figur 5 zeigt eine andere Ausführungsform des Innenliners 8, in dem sich im distalen Ende 2 eine Einlassöffnung 22 befindet. Der in Figur 5 gezeigte Innenliner 8 kann jedoch auch beispielsweise in einen Außenliner 6 oder in einen Prothesenschaft eingeführt werden, so dass die Einlassöffnung eine Verbindung mit dem sich zwischen dem Innenliner und dem jeweils anderen Bauteil, also dem Außenliner oder dem Prothesenschaft, befindlichen Zwischenvolumen bildet, wobei das Zwischenvolumen als Pumpkammer fungiert. Die Einlassöffnung 22 ist mit dem Strömungskanal 16 verbunden, der im Inneren des Innenliners 8 in Richtung auf das proximale Ende 4 des Innenliners 8 geführt ist. Man erkennt oberhalb des Strömungskanals 16 zwei Folienelemente 18, die in ihrem unteren Bereich mit dem Strömungskanal 16 verbunden sind und in ihrem oberen Bereich aneinander anliegen. Wird nun Luft von der Einlass-öffnung 22 durch den Strömungskanal 16 gepumpt, werden die beiden Folienelemente 18 auseinandergedrückt und geben den Weg für die Luft nach oben, also in Richtung auf das proximale Ende 4 des Innenliners 8 frei. Am proximalen Rand des Außenliners befindet sich eine Auslassöffnung 24, durch die die Luft entweichen kann. Da die beiden Folienelemente 18 im gezeigten Ausführungsbeispiel großflächig aneinander anliegen, kann durch die Auslassöffnung 24 keine Luft in das System eindringen, so dass die Einlassöffnung 22 und das sich gegebenenfalls daran anschließende Volumen nicht belüftet werden kann.

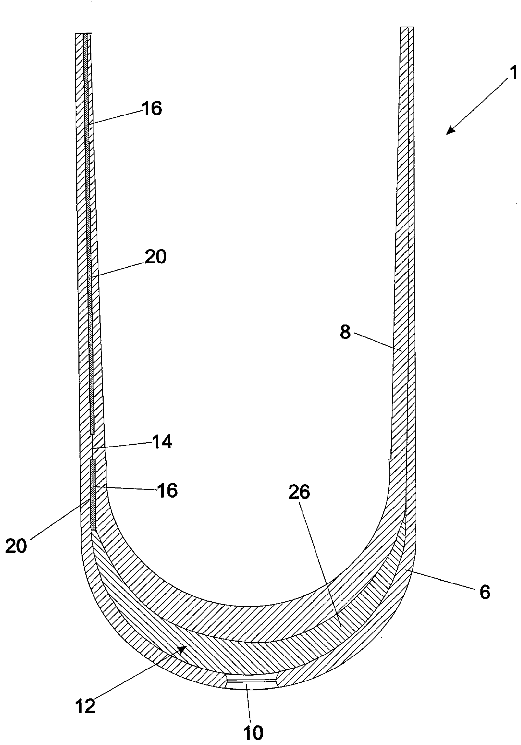

Figur 6 zeigt eine andere Ausgestaltung des Liners 1. Man erkennt den Innenliner 8 und den Außenliner 6, in dem sich die Einlassöffnung 10 befindet. Zwischen dem Innenliner 8 und dem Außenliner 6 befindet sich die Pumpkammer 12, die mit einem Schaumstoffmaterial 26 gefüllt ist. Durch dessen Elastizität wird gewährleistet, dass die Pumpkammer 12 wieder in ihre ursprüngliche Form zurückkehrt, nachdem sich durch einen äußeren Druck zusammengepresst wurde und der äußere Druck entfernt wurde. Dadurch wird gewährleistet, dass erneut Luft durch die Einlassöffnung 10 in die Pumpkammer 12 eindringen kann.

Der Strömungskanal 16 erstreckt sich in Figur 6 vom linken Ende der Pumpkammer 12 im Inneren zwischen dem Innenliner 8 und dem Außenliner 6 nach oben. Er ist beispielsweise in Form eines Schlauchs 20 gebildet und wird von einem Ein-Wege-Ventil 14 unterbrochen, das im gezeigten Ausführungsbeispiel recht kurz ausgebildet ist. Während im in Figur 5 gezeigten Ausführungsbeispiel die beiden Folienelemente 18 gemeinsam das Ein-Wege-Ventil 14 bilden, das sich nahezu über die gesamte Länge des Liners 1 erstreckt, ist das Ein-Wege-Ventil 14 im in Figur 6 gezeigten Ausführungsbeispiel sehr kurz ausgebildet. Dennoch sorgt es dafür, dass Luft nur aus der Pumpkammer 12 durch den Strömungskanal 16 nach außen strömen kann, während ein Einströmen von Luft durch den Strömungskanal 16 verhindert wird.

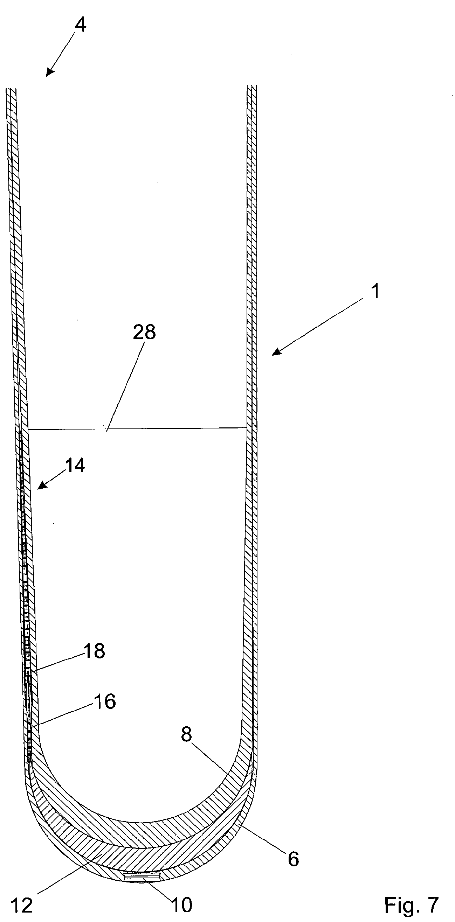

Auch in Figur 7 ist der Liner 1 mit dem Innenliner 8 und dem Außenliner 6 dargestellt, zwischen denen sich die Pumpkammer 12 mit der Einlassöffnung 10 befindet. Im linken Bereich befindet sich der Strömungskanal 16, der im oberen Bereich durch zwei Folienelemente 18 umgeben wird, die im weiteren Verlauf des Strömungskanals 16 aneinander anliegen und gemeinsam das Ein-Wege-Ventil 14 bilden.

Anders als im in Figur 5 gezeigten Ausführungsbeispiel verläuft der Strömungskanal 16 mit den beiden Folienelementen 18 jedoch nicht bis zum proximalen Ende 4 des Liners 1. Stattdessen endet der Strömungskanal 16 in etwa halber Höhe des Liners 1. Wird nun der in Figur 7 gezeigte Liner 1 angelegt, kann der Außenliner 6 bis in Höhe einer Umklappkante 28 umgeklappt werden und umgibt somit einen außerhalb des Außenliners 6 angeordneten Prothesenschaft. Dies sorgt einerseits dafür, dass es zwischen dem Außenliner 6 und dem Prothesenschaft, der in Figur 7 nicht dargestellt ist, zu einer luftdichten Abdichtung kommt, so dass ein Zwischenvolumen zwischen dem Außenliner 6 und dem Prothesenschaft durch die Pumpkammer 12 evakuiert werden kann, ohne dass Luft nachströmen kann.

Andererseits führt das Umklappen des Außenliners 6 dazu, dass Luft, die durch die Pumpkammer und den Strömungskanal 16 durch die beiden Folienelemente 18 gepresst wird, nun in Höhe der Umklappkante 28 den Liner 1 verlassen kann. Die Umklappkante 28 und insbesondere deren Position entlang des Liners 1 ist dabei nur schematisch dargestellt und nicht maßstabsgetreu.

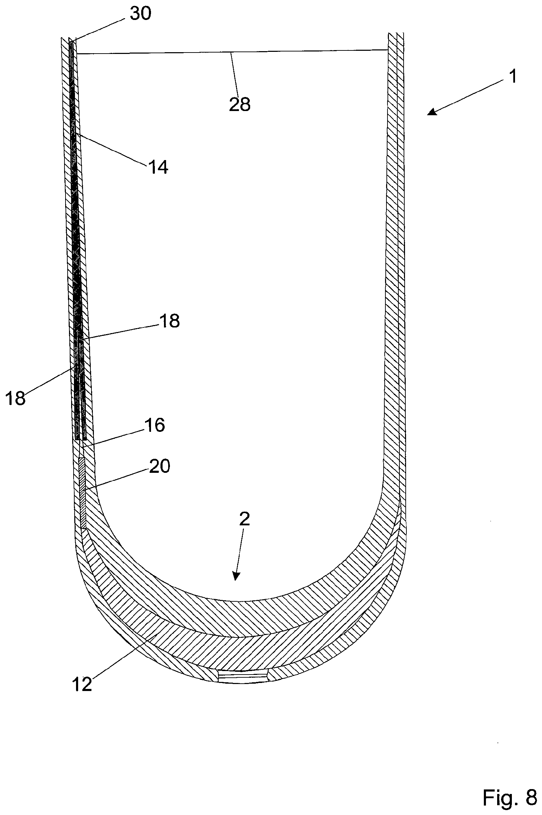

Figur 8 zeigt einen vergrößerten Ausschnitt aus Figur 7. Man erkennt den Strömungskanal 16, der im unteren Bereich, also zum distalen Ende 2 hin, durch einen Schlauch 20 gebildet wird. Daran schließen sich die beiden Folienelemente 18 an, die in Richtung auf das proximale Ende 4 des Liners 1 aneinander anliegen und so gemeinsam das Ein-Wege-Ventil 14 bilden. Der Innenliner 8 und der Außenliner 6 sind vorteilhafterweise vollflächig aneinander befestigt, um einen optimalen Halt der beiden Liner aneinander zu gewährleisten. Im gezeigten Ausführungsbeispiel gilt dies jedoch nur im distalen Bereich bis zur Umklappkante 28. Oberhalb der Umklappkante 28, die gegebenenfalls nur eine gedachte Linie ist, können die beiden Liner 6, 8 relativ zueinander bewegt werden. Insbesondere kann der Außenliner 6 umgeklappt werden und sorgt so für eine Abdichtung zwischen dem Außenliner 6 und dem nicht gezeigten Prothesenschaft. In diesem Fall befindet sich ein proximales Kanalende 30 am oberen Rand des Liners, so dass Luft aus der Pumpkammer 12 austreten kann. Alternativ oder zusätzlich zu dieser Lösung ist es bei Linern 1 gemäß den in Figur 7 und 8 gezeigten Ausführungsbeispielen auch möglich, Luft, die durch den Strömungskanal 16 und die beiden Folienelemente 18 durch das Ein-Wege-Ventil 14 gedrückt wird, am proximalen Kanalende 30 seitlich aus dem Liner 1 herauszuführen und gegebenenfalls durch eine Durchführung durch den Prothesenschaft abzuführen.

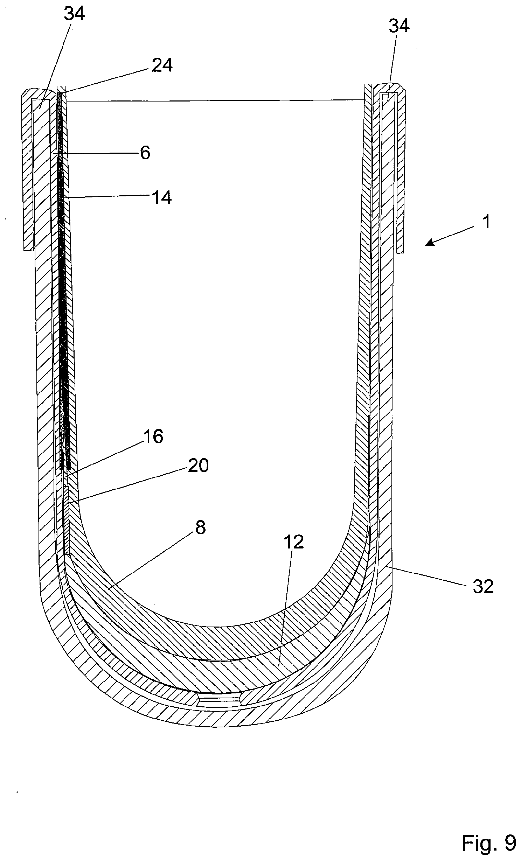

Figur 9 zeigt eine weitere Schnittdarstellung durch den Liner 1, mit Innenliner 8, Außenliner 6 und dazwischen angeordneter Pumpkammer 12 sowie dem Schlauch 20, dem Strömungskanal 16 und dem Ein-Wege-Ventil 14. Der Liner 1 entspricht dabei den in den Figuren 7 und 8 dargestellten Linern, ist in Figur 9 jedoch in einem Prothesenschaft 32 angeordnet dargestellt. Man erkennt, dass der Außenliner 6 über einen proximalen Rand 34 des Prothesenschaft 32 umgeklappt ist. Dadurch wird einerseits die Stabilität erhöht und andererseits die Auslassöffnung 24 freigegeben.

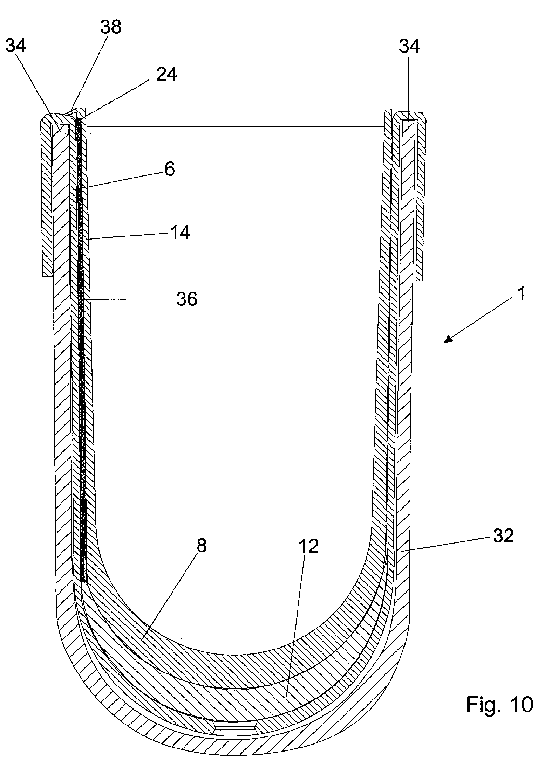

Figur 10 zeigt einen weiteren Liner 1 in einer Schnittdarstellung, der dem in Figur 9 gezeigten Liner 1 stark ähnelt. Auch er umfasst den Außenliner 6, den Innenliner 8 und die Pumpkammer 12 und ist im gezeigten Ausführungsbeispiel in den Prothesenschaft 32 eingesetzt. Der Außenliner 6 ist um den proximalen Rand 34 umgeklappt. Das Ventil 14 wird im gezeigten Ausführungsbeispiel jedoch durch eine zusätzliche Linerschicht 36 gebildet, die luftdurchlässig ausgebildet ist. Im belasteten Zustand wird die Pumpkammer 12 komprimiert, so dass Luft aus der Pumpkammer 12 durch die Linerschicht 36 im gezeigten Ausführungsbeispiel nach oben befördert werden kann. Die Auslassöffnung 24 ist dabei von einem Abschlusselement 38 abgedeckt, dass am Innenliner 8 angeordnet ist und so flexibel und elastisch ausgebildet ist, dass es von der durch das Ventil 14 strömenden Luft geöffnet werden kann.

Im entlasteten Zustand der Prothese dehnt sich die Pumpkammer 12 aus, so dass sich in ihr ein Unterdruck bildet. Ein Einströmen von Luft wird einerseits durch die Kompression der Linerschicht 36 durch den Umgebungsdruck bzw. durch den von dem Prothesenschaft 32 aufgebrachten Druck und andererseits durch das Abschlusselement 38 verhindert, dass die Auslassöffnung 24 verschließt.

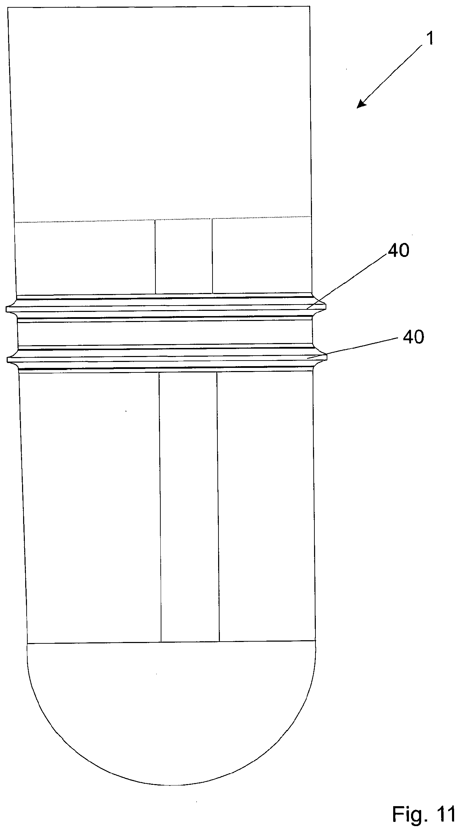

Der in Figur 11 gezeigte Liner 1 entspricht dem in Figur 4 dargestellten Liner, wobei in Figur 11 nun zwei Dichtlippen 40 um den Liner 1 herum angeordnet sind. Diese sorgen für einen dichtenden Kontakt zur Innenseite eines über den Liner 1 gezogenen Prothesenschaft.

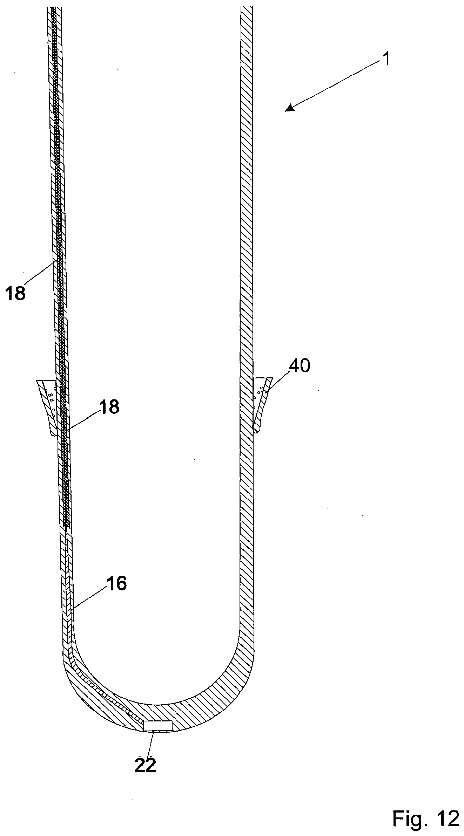

Der in Figur 12 gezeigte Liner 1 entspricht dem in Figur 5 gezeigten Liner, der über die Einlassöffnung 22 und den Strömungskanal 16 Luft aus dem Zwischenraum zwischen dem Liner 1 und einem darüber gezogenen Prothesenschaft herauspumpen kann. Dazu sind die beiden Folienelemente 18 vorhanden. Auch dieser Liner 1 verfügt nun über eine Dichtlippe 40, die von der Außenseite des Liners abstehend angeordnet ist. Im gezeigten Ausführungsbeispiel ist sie als separates Bauteil gefertigt und beispielsweise am Liner 1 angeklebt worden. Alternativ könnte sie jedoch auch als einstückiges Element mit dem Liner 1 hergestellt werden. Die Dichtlippe 40 sorgt für einen dichten Kontakt zum Prothesenschaft, der über den Liner 1 gezogen wird.

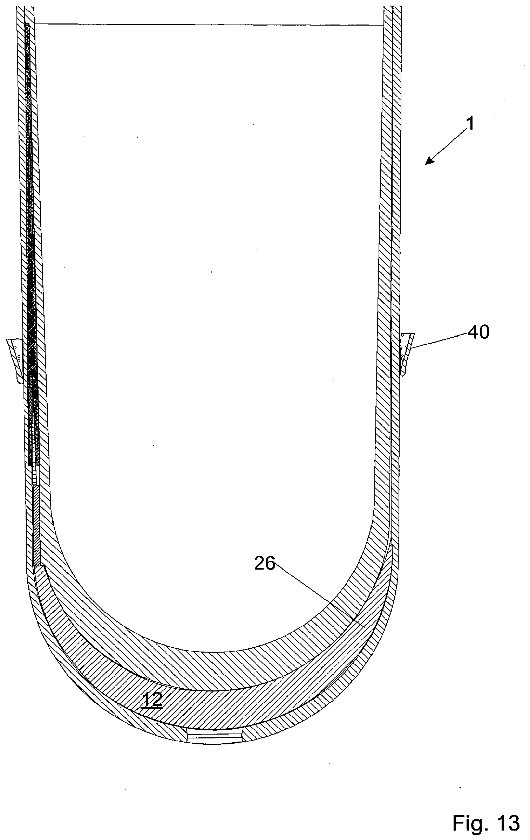

Der in Figur 13 gezeigte Liner 1 entspricht dem in Figur 6 gezeigten Liner und verfügt über eine Pumpkammer 12 aus dem Schaumstoffmaterial 26. Auch hier ist an der Außenseite des Liners eine Dichtlippe 40 angeordnet.

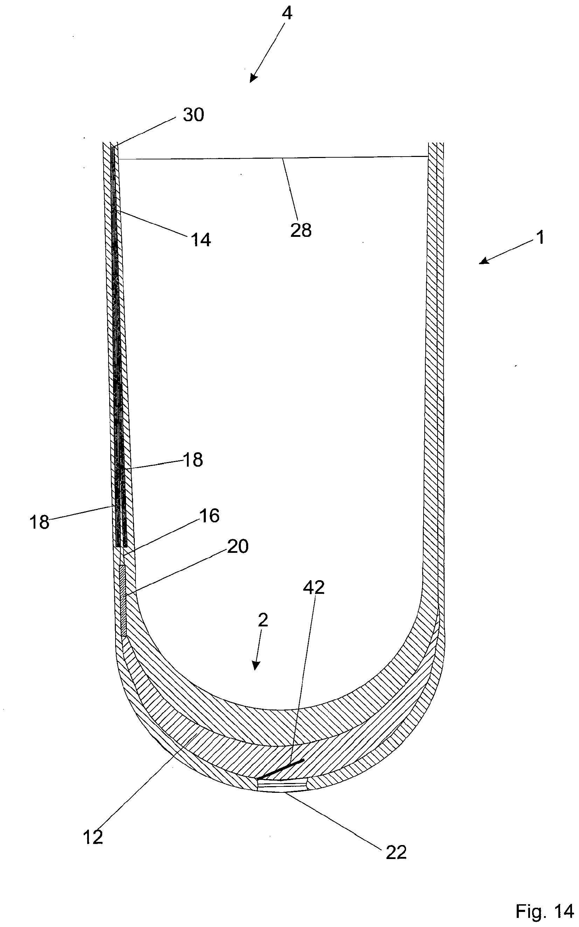

Der in Figur 14 gezeigte Liner 1 entspricht dem Liner aus Figur 8. Zusätzlich verfügt der in Figur 14 gezeigte Liner 1 jedoch über ein zweites Ein-Wege-Ventil 42, das an der Innenseite der Einlassöffnung 22 angeordnet ist. Dieses kann beispielsweise in Form eines einzelnen Folienelementes ausgebildet sein, das wie in Figur 14 dargestellt angeordnet ist. In der Standphase, wenn der Amputationsstumpf den Liner 1 in den Prothesenschaft 32 hineindrückt, verschließt das zweite Ein-Wege-Ventil 42 die Einlassöffnung 22 vollständig. In diesem Fall wird Luft, die sich in der Pumpkammer 12 befindet, durch das Ein-Wege-Ventil 14 und den Strömungskanal 16 ausgestoßen. In der Schwungphase wird der Prothesenschaft entlastet und das zweite Ein-Wege-Ventil 42 in die in Figur 14 gezeigte Position gebracht, so dass Luft durch die Einlassöffnung 22 in die Pumpkammer 12 eindringen kann.

### Bezugszeichenliste

- 1: Liner
- 2: Distales Ende
- 4: Proximales Ende
- 6: Außenliner
- 8: Innenliner
- 10: Einlassöffnung
- 12: Pumpkammer
- 14: Ein-Wege-Ventil
- 16: Strömungskanal
- 18: Folienelemente
- 20: Schlauch
- 21: poröses luftdurchlässiges Material
- 22: Einlassöffnung
- 24: Auslassöffnung
- 26: Schaumstoffmaterial
- 28: Umklappkante
- 30: Proximales Kanalende
- 32: Prothesenschaft
- 34: Proximaler Rand
- 36: Linerschicht
- 38: Abschlusselement
- 40: Dichtlippe
- 42: zweites Ein-Wege-Ventil

## Patentansprüche

1. Liner (1) für eine Prothese, der eine Innenseite und eine Außenseite aufweist, **wobei** zwischen der Innenseite und der Außenseite wenigstens ein Strömungskanal (16) mit wenigstens einer Einlassöffnung (10) und wenigstens einer Auslassöffnung verläuft, wobei ein Ein-Wege-Ventil (14) derart im Verlauf des wenigstens einen Strömungskanals (16) angeordnet ist, dass dieser nur von der wenigstens einen Einlassöffnung (10) zu der wenigstens einen Auslassöffnung durchströmbar ist, **dadurch gekennzeichnet, dass** zwischen der Innenseite und der Außenseite wenigstens eine Pumpkammer (12) in Fluidverbindung mit dem wenigstens einen Strömungskanal (16) angeordnet ist.

2. Liner (1) nach Anspruch 1, **dadurch gekennzeichnet, dass** der Liner (1) wenigstens abschnittsweise einen Innenliner (8) und einen Außenliner (6) aufweist.

3. Liner (1) nach Anspruch 2, **dadurch gekennzeichnet, dass** der Innenliner (8) und/oder der Außenliner (6) aus Polyurethan oder Silikon besteht.

4. Liner (1) nach einem der vorstehenden Ansprüche, **dadurch gekennzeichnet, dass** das Ein-Wege-Ventil (14) als Flatterventil ausgebildet ist.

5. Liner (1) nach einem der vorstehenden Ansprüche, **dadurch gekennzeichnet, dass** das Ein-Wege-Ventil (14) aus zwei Linerschichten gebildet ist.

6. Liner (1) nach Anspruch 5, **dadurch gekennzeichnet, dass** das Ein-Wege-Ventil (14) aus dem Innenliner (8) und dem Außenliner (6) gebildet ist.

7. Liner (1) nach Anspruch 5, **dadurch gekennzeichnet, dass** ein Ventilschnabel des Flatterventils aus mehreren Folienelementen (18) besteht.

8. Liner (1) nach Anspruch 7, **dadurch gekennzeichnet, dass** der Ventilschnabel aus zwei Folienelementen (18) besteht.

9. Liner (1) nach einem der vorstehenden Ansprüche, **dadurch gekennzeichnet, dass** die wenigstens eine Einlassöffnung (10) ein Loch in dem Außenliner (6) ist.

10. Liner (1) nach einem der vorstehenden Ansprüche, **dadurch gekennzeichnet, dass** die wenigstens eine Pumpkammer (12) eine Umfangswandung aus einem elastisch verformbaren Material aufweist.

11. Liner (1) nach Anspruch 10, **dadurch gekennzeichnet, dass** die Umfangswandung aus dem Innenliner (8) und dem Außenliner (6) gebildet ist.

12. Liner (1) nach einem der vorstehenden Ansprüche , **dadurch gekennzeichnet, dass** sich in der Pumpkammer (12) ein elastisch verformbares Material befindet.

13. Liner (1) nach Anspruch 12, **dadurch gekennzeichnet, dass** sich in der Pumpkammer (12) ein offenporiges Material befindet.

14. Liner (1) nach einem der vorstehenden Ansprüche, **dadurch gekennzeichnet, dass** der Innenliner (8) und der Außenliner (6) in einem proximalen Bereich (4) des Liners (1) so aneinander angeordnet sind, dass der Außenliner (6) separat vom Innenliner (8) umklappbar ist.

15. Liner (1) nach einem der vorstehenden Ansprüche, **dadurch gekennzeichnet, dass** die Einlassöffnung (10) sich distal und/oder die Auslassöffnung sich proximal am Liner (1) befindet.

## Claims

1. Liner (1) for a prosthesis, having an inner face and an outer face, wherein at least one flow channel (16) with at least one inlet opening (10) and at least one outlet opening extends between the inner face and the outer face, wherein a one-way valve (14) is arranged in the course of the at least one flow channel (16) in such a manner that medium can flow through it only from the at least one inlet opening (10) to the at least one outlet opening, **characterized in that,** between the inner face and the outer face, at least one pump chamber (12) is arranged fluidically connected to the at least one flow channel (16).

2. Liner (1) according to Claim 1, **characterized in that** the liner (1) has, at least in sections, an inner liner (8) and an outer liner (6).

3. Liner (1) according to Claim 2, **characterized in that** the inner liner (8) and/or the outer liner (6) is made of polyurethane or silicone.

4. Liner (1) according to one of the preceding claims, **characterized in that** the one-way valve (14) is designed as a flutter valve.

5. Liner (1) according to one of the preceding claims, **characterized in that** the one-way valve (14) is formed from two liner layers.

6. Liner (1) according to Claim 5, **characterized in that** the one-way valve (14) is formed from the inner liner (8) and the outer liner (6).

7. Liner (1) according to Claim 5, **characterized in that** a valve beak of the flutter valve is composed of several film elements (18).

8. Liner (1) according to Claim 7, **characterized in that** the valve beak is composed of two film elements (18).

9. Liner (1) according to one of the preceding claims, **characterized in that** the at least one inlet opening (10) is a hole in the outer liner (6).

10. Liner (1) according to one of the preceding claims, **characterized in that** the at least one pump chamber (12) has a peripheral wall made of an elastically deformable material.

11. Liner (1) according to Claim 10, **characterized in that** the peripheral wall is formed from the inner liner (8) and the outer liner (6).

12. Liner (1) according to one of the preceding claims, **characterized in that** an elastically deformable material is located in the pump chamber (12).

13. Liner (1) according to Claim 12, **characterized in that** an open-pore material is located in the pump chamber (12).

14. Liner (1) according to one of the preceding claims, **characterized in that** the inner liner (8) and the outer liner (6) are arranged on each other in a proximal area (4) of the liner (1) in such a manner that the outer liner (6) can be turned back separately from the inner liner (8).

15. Liner (1) according to one of the preceding claims, **characterized in that** the inlet opening (10) is located distally and/or the outlet opening is located proximally on the liner (1).

## Revendications

1. Doublure (1) pour une prothèse, comprenant un côté intérieur et un côté extérieur, dans laquelle au moins un canal d'écoulement (16) avec au moins une ouverture d'entrée (10) et au moins une ouverture de sortie s'étend entre le côté intérieur et le côté extérieur, une vanne unidirectionnelle (14) étant disposée dans le tracé dudit au moins un canal d'écoulement (16) de telle sorte que celui-ci ne peut être traversé que depuis ladite au moins une ouverture d'entrée (10) vers ladite au moins une ouverture de sortie,
**caractérisée en ce que**
au moins une chambre de pompage (12) est disposée en communication fluidique avec ledit au moins un canal d'écoulement (16), entre le côté intérieur et le côté extérieur.

2. Doublure (1) selon la revendication 1,
**caractérisée en ce que**
la doublure (1) comprend au moins localement une doublure intérieure (8) et une doublure extérieure (6).

3. Doublure (1) selon la revendication 2,
**caractérisée en ce que**
la doublure intérieure (8) et/ou la doublure extérieure (6) est constituée en polyuréthane ou en silicone.

4. Doublure (1) selon l'une des revendications précédentes,
**caractérisée en ce que**
la vanne unidirectionnelle (14) est réalisée sous forme de vanne à clapet.

5. Doublure (1) selon l'une des revendications précédentes,
**caractérisée en ce que**
la vanne unidirectionnelle (14) est réalisée par deux couches de doublure.

6. Doublure (1) selon la revendication 5,
**caractérisée en ce que**
la vanne unidirectionnelle (14) est formée par la doublure intérieure (8) et par la doublure extérieure (6).

7. Doublure (1) selon la revendication 5,
**caractérisée en ce que**
un bec de la vanne à clapet est constitué par plusieurs éléments en film (18).

8. Doublure (1) selon la revendication 7,
**caractérisée en ce que**
le bec de la vanne à clapet est constitué par deux éléments en film (18).

9. Doublure (1) selon l'une des revendications précédentes,
**caractérisée en ce que**
ladite au moins une ouverture d'entrée (10) est un trou dans la doublure extérieure (6).

10. Doublure (1) selon l'une des revendications précédentes,
**caractérisée en ce que**
ladite au moins une chambre de pompage (12) présente une paroi périphérique en un matériau élastiquement déformable.

11. Doublure (1) selon la revendication 10,
**caractérisée en ce que**
la paroi périphérique est formée par la doublure intérieure (8) et par la doublure extérieure (6).

12. Doublure (1) selon l'une des revendications précédentes,
**caractérisée en ce que**
un matériau élastiquement déformable se situe dans la chambre de pompage (12).

13. Doublure (1) selon la revendication 12,
**caractérisée en ce que**
un matériau à pores ouverts se situe dans la chambre de pompage (12).

14. Doublure (1) selon l'une des revendications précédentes,
**caractérisée en ce que**
dans une zone proximale (4) de la doublure (1), la doublure intérieure (8) et la doublure intérieure (6) sont disposées l'une contre l'autre de telle sorte que la doublure extérieure (6) peut être rabattue séparément de la doublure intérieure (8).

15. Doublure (1) selon l'une des revendications précédentes,
**caractérisée en ce que**
l'ouverture d'entrée (10) se trouve en situation distale et/ou l'ouverture de sortie se trouve en situation proximale sur la doublure (1).
